# EUROPEAN PATENT APPLICATION

(11) **EP 3 656 289 A1**
(43) Date of publication of application: **27.05.2020**
(21) Application number: 18835918.6
(22) Date of filing: 17.07.2018
(51) Int. Cl.: A61B 5/00, A61B 3/113

(54) **BRAIN FUNCTION TESTING SYSTEM AND DEVICE THEREOF**

(30) Priority: 21.07.2017 CN 201710599005
(71) Applicant: Jasmines Biotech Inc., Beijing 100195 (CN)
(72) Inventor: ZHANG, Mingsha, Beijing 100875 (CN); WU, Si, Beijing 100875 (CN); CHU, Dianhui, Chengdu Sichuan 610200 (CN); LIN, Xiaohan, Beijing 100081 (CN)
(74) Representative: Goulard, Sophie
(86) International application number: PCT/CN2018/095922
(87) International publication number: WO 2019/015567

(57) **Abstract**

The invention discloses a method to test brain function based on measuring saccadic eye movement, which comprises a plurality of modules of eye movement tasks, eye movement data collection and data storage and analysis module. Among them, real-time analysis of head and eye position data is used in eye movement data collection to compensate for the impact of head movement on eye position, and the result is more accurate. The invention also discloses a device using the system, which can be used for determining various neurodegenerative diseases.

## Description

### Priority Date Claimed

This application is a Section 371 National Stage of international application PCT/CN2018/095922, filed July 17, 2018, and claims the benefit of priority of Chinese Patent Application Serial No. 201710599005.4, filed on July 21, 2017, the entire content of which is incorporated by reference herein.

### Technical field

The present invention relates generally to the field of life sciences and, in particular, to a system and its device for detecting brain function in the field of neurobiology.

### Background technology

Neurodegenerative diseases often only show clinical symptoms when the number of damaged neural cells in brain reaches a certain level. Alzheimer's Disease being an example, the onset of the Alzheimer's Disease is slow or hidden, and patients and family members often do not recall the onset. It is more common in elderly people aged over 70 (with an average age of male patients of 73 and an average age of female patients of 75). A minority of patients' symptoms quickly become clear after physical illness, fracture, or mental stimulation. Female patients outnumber male patients (female : male ratio is 3 : 1). Main manifestations are decreased cognitive function, mental symptoms and behavioral disorders, and gradual decline in daily self-care ability.

To date, the pathogenesis of neurodegenerative diseases has not been clearly understood, no convenient methods are available to collect reliable biomarkers to help diagnoses, and no effective treatments are available to cure the diseases despite many efforts made in the fields of medicine and neuroscience. Therefore, early diagnosis and early intervention of neurodegenerative diseases are of great significance. Early detection and prevention in high-risk populations will delay the progression of the diseases, improve patients' life expectancy and life quality, and alleviate family and social burdens.

The current diagnosis of neurodegenerative disorders depends on the following methods: 1) Charting method or interview method. Although this method is easy to operate, the charting or interview is often not sufficiently objective and comprehensive; 2) Biomarker detection. For example, β amyloid and Tau proteins are abnormally aggregated proteins in the brains of patients with Alzheimer's disease. Clinically, observing the contents of β amyloid and tau proteins in the cerebrospinal fluid can help diagnosis of Alzheimer's Disease. Because the test specimen is cerebrospinal fluid which can only be obtained by lumbar puncture, the test is difficult to operate and might cause great damages, therefore it is difficult to be widely duplicated or applied; 3) Neuroimaging test. With the development of neuroimaging technologies, brain imaging methods such as MRI and PET have been applied to the diagnosis of neurodegenerative diseases in recent years. However, this method can only be performed in hospitals where MRI or PET equipment are available, and such methods are expensive and difficult to popularize and apply. More importantly, although MRI is a non-invasive and harmless examination method, MRI brain images cannot provide diagnostic clues during the early stages of the neurodegenerative diseases when there are no anatomical changes. On the other hand, although brain function tests using PET can detect changes in brain functions, PET brain imaging requires intravenous injection of radioactive agents and is an invasive test, making it difficult to convince patients to cooperate with the test before clinical symptoms appear.

In summary, early diagnosis of neurodegenerative diseases and early detection of high-risk populations urgently require a safe, reliable, universal, and inexpensive examination method. Based on our long-term scientific research findings, we designed a brain function testing device that provides reliable physical indicators for the diagnosis of neurodegenerative diseases by observing changes in eye movement parameters. The design principle of the brain function test device is based on the behavioral and cognitive dysfunction of patients caused by neurodegenerative diseases in the early stage, such as decreased memory and attention, and sensory motor abnormalities. More importantly, these changes in behavior and cognitive function can even appear before abnormal brain function images.

Saccadic eye movements are the most common autonomous movements among the five types of human eye movements. Saccades are controlled by multiple brain regions (such as the prefrontal cortex, posterior parietal cortex, visual cortex, basal ganglia, cerebellum, superior colliculus, thalamus, etc.) and their neural circuits. Saccades are closely related to the condition of cognitive functions (such as memory, attention, etc.). Under many neuropathological conditions, the saccade patterns often undergo changes, so the patterns can be used as support for clinical disease diagnosis.

### Summary of the Invention

One aspect of the present invention provides a saccade analysis system based on detecting brain function, the system comprises:
a vision and saccade task module, the module comprising one or more of the following tasks:
1) Pro-saccade task: The subject first looks at the gaze (fixation) point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
4) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
Pro-saccade task: The subject first looks at the gaze point, and then when the visual stimulus point appears, the subject is tasked to make a quick saccade toward the visual stimulation point;

In a specific embodiment of the invention, the vision and saccade task module comprises:
Anti-saccade task: The subject first looks at the gaze point, and then when the visual stimulus point appears, the subject is tasked to make a quick saccade toward the mirror position of the visual stimulation point.

In a specific embodiment of the invention, the vision and saccade task module comprises:
Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.

In a specific embodiment of the invention, the vision and saccade task module comprises:
Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Anti-saccade task: The subject first looks at the gaze point, and then when the visual stimulus point appears, the subject is tasked to make a quick saccade toward the mirror position of the visual stimulation point;
2) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.

In a specific embodiment of the invention, the vision and saccade task module comprises:
1) Anti-saccade task: The subject first looks at the gaze point, and then when the visual stimulus point appears, the subject is tasked to make a quick saccade toward the mirror position of the visual stimulation point;
2) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
2) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared;
3) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Anti-saccade task: The subject first looks at the gaze point, and then when the visual stimulus point appears, the subject is tasked to make a quick saccade toward the mirror position of the visual stimulation point;
2) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
3) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the vision and saccade task module comprises:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
4) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.

In a specific embodiment of the invention, the aforementioned gaze point and visual stimulus points do not overlap.

The four saccade tasks described above have different emphases on the detection of the physiological and cognitive functions of the brain. Among them, pro-saccade task is the simplest and is mainly used to detect basic vision-eye movement information processing. If abnormal, it indicates that the function of cortical and subcortical vision-eye movement neural circuit (including the primary visual cortex) is abnormal. The anti-saccade task is mainly used to detect the brain's ability to suppress reflex response. If abnormal, it indicates that the cerebral cortex (mainly prefrontal cortex) of the subject is functionally abnormal. The memory-saccade task is mainly used to detect the short-term (working) memory ability of the brain. If abnormal, it indicates that the subject's cerebral cortex (mainly the prefrontal lobe, posterior parietal lobe, hippocampus, etc.) is functionally abnormal. The double-saccade task is mainly used to test the spatial cognitive ability of the brain. If abnormal, it indicates that the subject's cerebral cortex (mainly the posterior parietal lobe, prefrontal lobe, hippocampus, etc.) is functionally abnormal. The vision and saccade task modules comprising the above four types of saccade tasks can explore the brain function of the subjects from multiple aspects. In the early stage of degenerative diseases of the brain, the damage to the neural cells of the brain and to the structure of the neural network is slight, so the effects on the function of the brain are mainly manifested in complex cognitive activities, while the effects on the basic physiological functions of the brain are small. In the data analysis, in addition to comparing the eye movement parameters of the subjects of the same age under the same eye movement task and identifying the subjects with abnormal eye movements, we also took the eye movement parameters during the pro-saccade task of each subject as the baseline of that subject, which is compared with the same eye movement parameters in the other three tasks, and the relative changes of each subject's eye movement parameters could reflect the specific cognitive function of the brain. By analyzing and comparing the execution of the above-mentioned four types of saccade tasks (for example, error rate, saccade latency, saccade velocity, saccade accuracy), the subjects' vision, movement, and cognitive status can be understood. These saccade tasks can accurately distinguish normal subjects from patients with neurodegenerative diseases. More importantly, through the multi-aspect exploration of the vision and saccade task modules comprising the aforementioned saccade tasks, it is possible to predict early stage brain dysfunctions such as neurodegenerative diseases earlier and more accurately. This is more sensitive than the physical examination or screening system currently in clinical use.

In one embodiment of the invention, the system as described above also comprises at least one of the following modules:
a) behavior data collection module;
b) data storage and analysis module;

In one embodiment of the invention, the aforementioned behavior data collection module comprises:
a1) using computer object recognition and tracking methods to process in real-time image information collected by fast near-infrared camera in order to identify pupils and detect eye positions;
a2) eliminating in real-time the effect of head movements on the eye position monitoring.

In a specific embodiment of the invention, the frequency of the aforementioned fast near-infrared camera is not lower than 1000 Hz.

In an embodiment of the present invention, the aforementioned method for eliminating in real-time the effect of head movements on the eye position:

Placing the inclinometer at the rotation point of the subject's head. When saccade and head rotation occur at the same time, the inclinometer records the current eye movement angle β and the head movement angle γ. The true saccade angle α can be obtained by subtracting γ from β.

In a specific embodiment of the present invention, the response frequency of the inclinometer is not lower than 200 Hz.

In an embodiment of the present invention, the aforementioned data storage and analysis module comprises:
b1) establishing saccade database based on saccade behavior paradigms;
b2) establishing a computational model and analyzing eye movement parameters.

Wherein, the eye movement parameters comprise at least one of error rate of saccadic traces, saccade latency, saccade velocity, and saccade accuracy.

The range of eye movement parameters is described in the following table:

| Parameter | Normal Control | Patient population with degenerative diseases |
|---|---|---|
| Error rate: | | |
| Pro-saccade task | <20% | >30% |
| Anti-saccade task | <40% | >50% |
| Memory-saccade task | <35% | >50% |
| Double-saccade task | <25% | >50% |

In another aspect of the present invention, a brain function detecting device is provided, which is characterized by utilizing any one of the aforementioned systems .

In an embodiment of the present invention, the brain function detecting device uses an eye movement analysis system based on brain function, and the system comprises:
vision and saccade task module, this module comprises:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
4) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared.
wherein the gaze point and the stimulus points do not overlap.

In an embodiment of the present invention, the vision and saccade task modules of the brain function detection device may be integrated in a behavior data collection device or may be integrated in a separately implemented device.

In an embodiment of the present invention, the above mentioned brain function detecting device further comprises at least one of the following modules:
a) behavior data collection module;
b) data storage and analysis module.

In an embodiment of the present invention, the behavior data collection module may be integrated into a behavior data collection device.

In an embodiment of the present invention, the behavior data storage and analysis module may be separately integrated in a data analysis device or may be integrated in a data collection device.

In one embodiment of the present invention, the behavior data collection module comprises:
a1) using computer object recognition and tracking methods to process in real-time image information collected by fast near-infrared camera in order to identify pupils and detect eye positions;
a2) eliminating in real-time the effect of head movements on the eye position monitoring.

In one embodiment of the present invention, the aforementioned behavior data collection device comprises:
a1) near-infrared fast imaging eye position detection device: real-time and quick identification and tracking eyes from images;
a2) inclinometer: real-time detection of the head position signal for the purpose of compensating for eye position changes caused by head movements.

In a specific embodiment of the invention, the frequency of the aforementioned fast near-infrared camera is not lower than 1000 Hz.

In an embodiment of the present invention, the aforementioned method for eliminating in real-time the effect of head movements on the eye position:
Placing the inclinometer at the rotation point of the subject's head. When saccade and head rotation occur at the same time, the inclinometer records the current eye movement angle β and the head movement angle γ. The true saccade angle α can be obtained by subtracting γ from β

In a specific embodiment of the present invention, the response frequency of the inclinometer is not lower than 200 Hz. In an embodiment of the present invention, the aforementioned data storage and analysis module or data analysis device comprises:
b1) establishing saccade database based on saccade behavior paradigms;
b2) establishing a computational model and analyzing eye movement parameters.

In a specific embodiment of the present invention, the aforementioned data storage and analysis module or data analysis device comprises: transmitting the collected eye position data to a computer server through a network, performing data analysis through computer modeling with the support of big data, establishing and improving reliable eye movement parameter standards to achieve the goal of early warning of brain function abnormalities.

In a specific embodiment of the invention, the aforementioned devices are used to monitor or detect brain function or neurodegenerative diseases.

In a specific embodiment of the present invention, the neurodegenerative diseases comprise Alzheimer's Disease of various severity, amyotrophic lateral sclerosis, Huntington's Disease, cerebellar atrophy, multiple sclerosis, Parkinson's Disease.

Another aspect of the present invention also provides a disease detection method for evaluating a subject's brain function based on saccades, which is characterized in that the method uses the following saccade behavior paradigm:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the circle centered at the gaze point, and disappears immediately. After a period of time when the gaze point disappears, the subject is tasked to make a quick saccade to the position where the visual stimulus point once appeared.
4) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared;
wherein the gaze point and the stimulus points do not overlap.

In an embodiment of the present invention, the method described above further comprises at least one of the following steps:
a) behavior data collection steps;
b) data storage and analysis steps.

In an embodiment of the present invention, the aforementioned behavior data collection module comprises:
a1) using computer object recognition and tracking methods to process in real-time image information collected by fast near-infrared camera in order to identify pupils and detect eye positions;
a2) eliminating in real-time the effect of head movements on the eye position monitoring.

In a specific embodiment of the invention, the frequency of the aforementioned fast near-infrared camera is above 1000 Hz.

In an embodiment of the present invention, the aforementioned method for eliminating in real-time the effect of head movements on the eye position:

Placing the inclinometer at the rotation point of the subject's head. When saccade and head rotation occur at the same time, the inclinometer records the current eye movement angle β and the head movement angle γ. The true saccade angle α can be obtained by subtracting γ from β.

In a specific embodiment of the present invention, the response frequency of the inclinometer is above 200 Hz.

In an embodiment of the present invention, the aforementioned data storage and analysis module described above comprises:
b1) establishing saccade database based on saccade behavior paradigms;
b2) establishing a computational model and analyzing eye movement parameters.

In a specific embodiment of the present invention, the aforementioned evaluating the brain function of a subject comprises diagnosing or assisting in diagnosing whether the subject has a neurodegenerative disease, wherein the neurodegenerative disease comprises different degrees of severity, such as mild, moderate, and severe; the neurodegenerative disease comprises early, intermediate, and terminal stages.

The neurodegenerative diseases include Alzheimer's disease, amyotrophic lateral sclerosis, Huntington's disease, cerebellar atrophy, multiple sclerosis, Parkinson's disease.

### Beneficial effects of the present invention

1. The invention discloses a system for detecting and analyzing eye movement parameters in a specific behavior paradigm which can be used for early detection of high-risk populations of brain degenerative diseases.
2. The invention also discloses a method for eliminating the effect of head movements on detecting eye position. By detecting head and eye position in real-time and synchronously and using computer modeling to integrate head and eye position information, the effect of eliminating the effect of head movements on eye position detection is achieved.
3. A detection method for early evaluation and screening of neurodegenerative diseases is disclosed, which can evaluate the treatment effect of neurodegenerative diseases and determine the choice of the best treatment plan and provide objective indicators for clinicians to help individualized and precise treatment (medicine, radiation therapy, etc.).
4. The disclosed brain function detection device can guide the training of cognitive functions, in order to achieve the purposes of physical therapy and delayed and improved disease conditions.

### Brief description of the figures

FIG. 1 shows a diagram showing the structural principles of a saccade analysis system based on brain function in the present invention.
FIG. 2 shows the pro-saccade task. The subject first looks at the gaze point (cross), and then when the visual stimulus point (dot) appears, the subject is tasked to make a quick saccade toward the visual stimulus point, whereby the control function of the subject's brain (for example, basal ganglia, superior colliculus) on the reflective saccade is examined.
FIG. 3 shows the anti-saccade task. The subject first looks at the gaze point (cross), and then when the visual stimulus point appears, the subject is tasked to make a quick saccade to the mirror position of the visual stimulus point (dot), whereby the brain's (e.g., prefrontal cortex, basal ganglia) inhibition of reflective saccades and ability to generate and control correct saccades are examined.

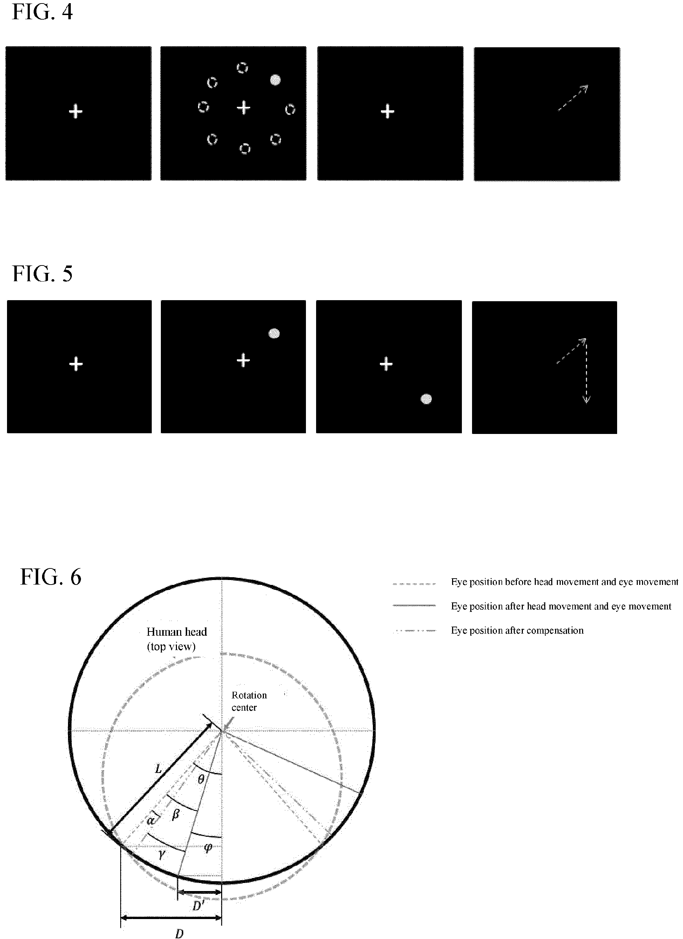

FIG. 4 illustrates the memory-saccade task. The subject first looks at the gaze point (cross), and then a visual stimulus point (dot) appears at a random position, selected among eight positions evenly distributed with same eccentricity centered at the gaze point, and disappears immediately. After a period of time when the gaze point disappears, the subject is tasked to make a quick saccade to the position where the visual stimulus point once appeared, whereby the spatial memory ability of the subject is examined, and the accuracy of the spatial memory of the subject's brain (for example, the frontal parietal cortex) is determined by analyzing the accuracy of the saccade.
FIG. 5 shows the double-saccade task. The subject first looks at the gaze point (cross). Then the two visual stimulus points (dots) sequentially appear and disappear. After the gaze point disappears, the subject is tasked to make two saccades to sequentially, in the order of appearance, look at the two positions where the visual stimulus points appeared whereby the ability of the subject's brain (for example, the posterior parietal cortex) to convert visual spatial information is measured.
FIG. 6 shows a block diagram showing the working principle of the brain function testing device of the present invention.
FIG. 7 shows a working principle diagram of the behavior data collection device.
FIGS. 8A through 8D show saccadic traces of normal subjects under pro-saccade tasks.
FIGS. 9A through 9H show saccadic traces of subjects with Parkinson's Disease (PD) under pro-saccade tasks.
FIGS. 10A through 10E show saccadic traces of subjects with mild cognitive impairment (MCI) and Alzheimer's Disease (AD) under pro-saccade tasks.
FIGS. 11A through 11D show saccadic traces of normal subjects under anti-saccade tasks.
FIGS. 12A through 12G show saccadic traces of subjects with Parkinson's Disease under anti-saccade tasks.
FIGS. 13A through 13E show saccadic traces of subjects with mild cognitive impairment and Alzheimer's Disease under anti-saccade tasks.
FIGS. 14A through 14D show saccadic traces of normal subjects under memory - saccade tasks.
FIGS. 15A through 15D show saccadic traces of subjects with Parkinson's Disease under memory-saccade tasks.
FIGS. 16A through 16E show saccadic traces of subjects with mild cognitive impairment and Alzheimer's Disease under memory-saccade tasks.
FIGS. 17A through 17D show saccadic traces of normal subjects under double-saccade tasks.
FIGS. 18A through 18F show saccadic traces of subjects with Parkinson's Disease under double-saccade tasks.
FIGS. 19A through 19D show saccadic traces of subjects with mild cognitive impairment and Alzheimer's Disease under double-saccade tasks.
FIG. 20 shows the group mean error rates under the four tasks of subjects in the normal, PD, and AD groups.
FIGS. 21A and 21B show the saccadic traces of two subjects (normal, suspected patient) under the pro-saccade task.
FIGS. 22A and 22B show the saccadic traces of two subjects (normal, suspected patient) under the anti-saccade task.
FIGS. 23A and 23B show the saccadic traces of two subjects (normal, suspected patient) under the memory-saccade task.
FIGS. 24A and 24B show the saccadic traces of two subjects (normal, suspected patient) under the double-saccade task.
FIGS. 25A through 25D show the saccadic traces under the saccade tasks of the present invention of a subject that is determined to be normal by the classic methods. The saccade tasks of the present invention indicate that the subject's saccadic traces are abnormal, and after detailed clinical review, the subject is diagnosed with essential tremor.
FIGS. 26A through 26D show the saccadic traces under the saccadic tasks of the present invention of a subject that is determined to be normal by the classic methods. The saccade tasks of the present invention indicate that the subject's saccadic traces are abnormal, and after detailed clinical review, the subject is diagnosed with mild cognitive impairment.
FIG. 27 shows the error rates of the normal group subjects and suspected patients under the four tasks.
FIG. 28 shows the accuracy of the normal group subjects and suspected patients under the pro-saccade task.
FIG. 29 shows the accuracy of the normal group subjects and suspected patients under the anti-saccade task.

### Detailed working description

The present invention is further described below through specific working embodiments and experimental data. Although technical terms are used hereinafter for clarity, these terms are not meant to define or limit the scope of the invention.

As used herein, the term "subject" comprises any human or non-human animal. The term "non-human animal" comprises all vertebrates, for example, mammals and non-mammals, such as non-human primates, sheep, dogs, cats, horses, cattle, chickens, amphibians, reptiles, etc. Unless specifically stated, the terms "patient" and "subject" are used interchangeably.

The terms "treatment" and "treatment method" refer to both therapeutic treatment and preventative measures. Those in need of treatment include individuals who already have a specific medical condition, as well as those who may eventually acquire the condition.

The term "neurodegenerative disease" refers to a disease state in which neurons of the brain and spinal cord are lost. Neurodegenerative diseases are caused by the loss of neurons or their myelin sheaths, where the diseases deteriorate over time to cause dysfunction. Neurodegenerative diseases are divided into two groups according to phenotype: one group affects motor movement, such as cerebellar ataxia; the other group affects memory and includes related dementia.

The term "eye movement" refers to eye movements, including but not limited to the eye staying on the processed object (called "gaze"), the rapid movement of the eye between two gaze points or between gaze point and stimulus point (called "saccade"), and the eye movement following a moving object (called "following movement"). The eye movement parameters of the present invention include, but are not limited to, at least one of error rate of saccadic traces, saccade latency, saccade velocity, and saccade accuracy.

The term "saccade" is a type of eye movements, which is the rapid movement of the eye between two gaze points or between a gaze point and a stimulus point.

Unless otherwise specified, the experimental methods in the following Working Examples are conventional methods.

### Working Examples:

### Example 1: A saccade analysis system and device based on brain degenerative diseases

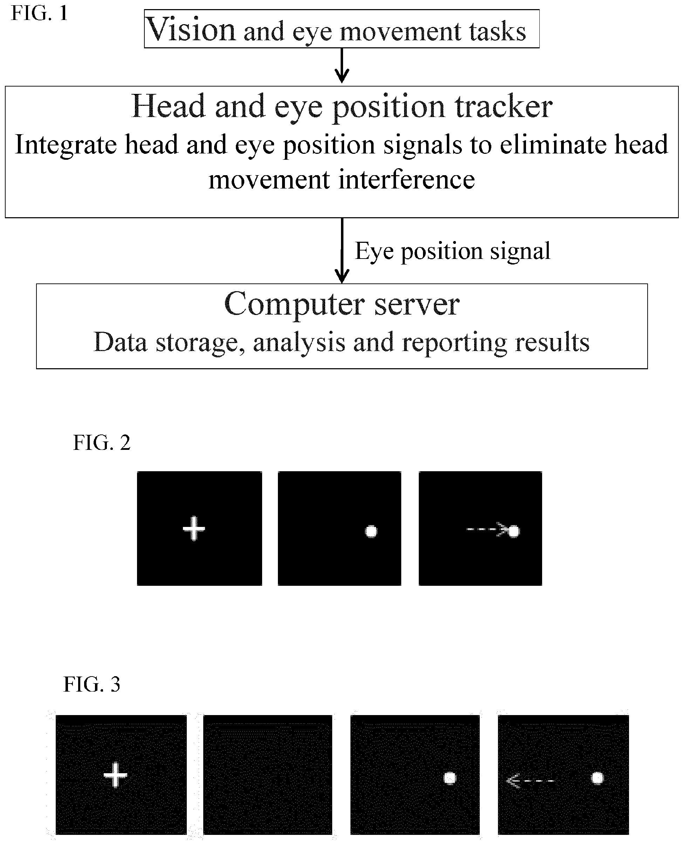

The example specifically comprises the following modules (FIG. 1)
1) Experimental environment control module or device: to eliminate the impact of the experimental environment (including light brightness, noise level, etc.) on saccades;
2) A head fixation device may be further included to avoid large movements of the subject's head during data collection;
3) Vision and saccade task module or device: to collect eye and head position signals and pre-process them under specific behavior tasks (FIG. 2 through 5);
4) Data collection module or device: for real-time collection of eye position signals through a near-infrared fast camera (1000 Hz), real-time collection of head position signals (200 Hz) through a head-mounted inclinometer, real-time analysis and processing of head, position signals, and compensation for the effect of head movement on eye positions (FIG. 3). The adopted calculation method is: the inclinometer needs to be placed on the pivot point of the head as much as possible. When saccade and head rotation occur simultaneously, the current eye movement angle β is recorded. Both head movements and saccades contribute to β. The head movement angle γ is obtained by the inclinometer. The true saccade angle α can be obtained by subtracting γ from β (FIG. 6);
5) Data processing and analysis module or device: real-time processed eye position signals and behavioral task codes are transmitted to a computer server through a network and added to a behavioral database; a computational model is established with the support of big data, and eye movement parameters are analyzed. Analysis are then returned to the user.

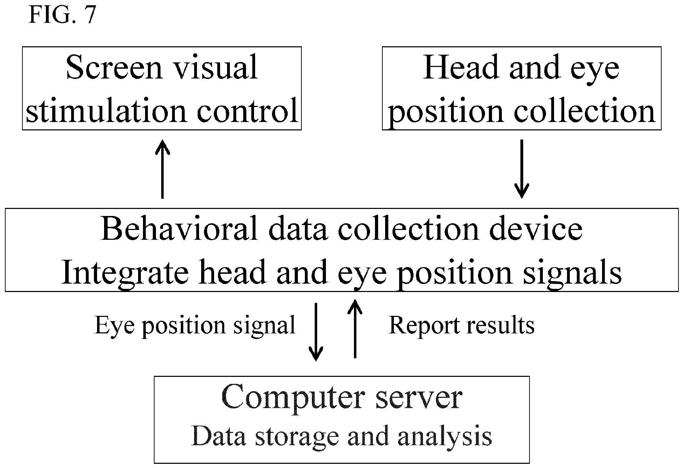

The working principle of a device prepared using this system is shown in FIG. 7. Behavioral data collection instrument sends screen visual stimulation signals (FIGS. 2 through 5). The data collection device (behavioral data collection instrument) collects eye position signals in real-time through a near-infrared fast camera (1000 Hz) and acquires head position signals in real-time through a head-mounted inclinometer (200 Hz) to analyze and integrate the head position signal in real time and compensate for the impact of head movement on the eye position. The processed eye position signal is transmitted to a computer server for data storage and analysis.

Current representative instruments based on eye movements to test brain function are these following:
1. Neurotrack Technologies Inc., a start-up located in Redwood City, California, has launched a system based on examining cognitive ability through monitoring the spatial pattern of gaze (fixation) during freely viewing a picture. Specifically, through a browser application program the system has the subjects freely observe pictures on a screen, the user's eyeballs moving accordingly, thereby checking whether the user's cognitive ability shows any signs of decline.
2. The DEM-200 eye movement detector produced by Shanghai Dikang Medical Biotechnology Co., Ltd. helps to diagnose mental illnesses clinically by detecting the spatial pattern of gaze (fixation) during freely viewing a picture and pupil changes of the subjects.

The similarity of the two systems described above is to determine the subject's cognitive ability (for example, memory ability) by detecting eye movement traces (gaze points) when viewing pictures freely. The system of the present invention observes saccades under a specific behavioral task paradigm, and determines physiological and cognitive functions of the brain by analyzing saccade parameters (such as saccade latency, saccade velocity, saccade accuracy, etc.).

**Table 1. Eye movement features of different neurodegenerative diseases**

| Neurodegenerative disease | Clinical eye movement characteristics | Laboratory eye movement characteristics |
|---|---|---|
| PD | Slightly insufficient upward amplitude of voluntary eye movement; Slightly impaired smooth pursuit | Decreased voluntary eye movement gain (such as insufficient amplitude) |
| PD with dementia | | Increased eye movement velocity; decreased reflex and voluntary eye movement gain; increased reverse eye movement error |
| HD patients | Difficult initiation of eye movements; slow eye movements (young patients); fixation drifting and not continuous | Increased eye movement velocity and variability; reduced eye movement velocity; increased eye movement direction and precise time error rate of reverse eye movement and memory-guided eye movement; smooth pursuit drifting |
| HD potential population | Normal | Increased eye movement velocity and variability; reduced eye movement velocity; increased eye movement direction and precise time error rate of anti-saccade and memory-saccade |
| AD patients | Visual fixation defect in anti-saccade test | Increased fixation instability; increased reflex and voluntary eye movement velocity; increased anti-saccade errors; decreased anti-saccade error correction |

Table 1 shows the eye movement characteristics of different neurodegenerative diseases. It is apparent that changes in eye movements are caused by Alzheimer's Disease, Parkinson's Disease, and Huntington's Disease. But the core issue of the present invention is what specific eye movement paradigm behavior combinations can be used to determine these diseases. Paradigm behavior combinations are massive, but the specific paradigm behaviors invented by the inventors can well determine these diseases. According to the specific eye movement features of degenerative diseases, the inventor organically combined pro-saccade task, anti-saccade task, memory-saccade task and double-saccade task, and the inventors found that this invention not only applies well to patients who have been identified clinically, but also identifies more sensitively potential patients with deficient brain function who are clinically determined to be normal.

### Example 2: Eye movement parameters of Alzheimer's Disease and Parkinson's Disease patients:

By using the saccade analysis system and device of the present invention, it is verified that the system can accurately distinguish between patients with neurodegenerative diseases and normal people:
(1) Different neurodegenerative diseases can cause specific changes in eye movement parameters in the four aforementioned behavioral paradigms. As follows:
   Alzheimer's Disease (AD): Increased gaze instability; increased saccade latency and variability; decreased saccade velocity, increased error rate of saccade direction and precise time of anti-saccade task and memory-saccade task; decreased anti-saccade correction.
   Parkinson's Disease (PD): Decreased voluntary eye movement gain (such as insufficient stretching); there are obvious defects in the initiation of memory-saccades and anti-saccades; inhibition of saccade amplitude can be detected early in PD, and may be related to basal ganglia function; but elongation of eye movement velocity usually occurs in the late stage of the disease, and is related to the impairment of cognitive ability, which may be caused by the impairment of the function of the diffuse non-dopamine system in the late stage of the disease.
   Huntington's Disease (HD): Increased saccade velocity and variability; decreased saccade velocity, increased error rate of saccade direction and precise time of anti-saccade task and memory-saccade task; vertical saccades are more prone to be affected than horizontal saccades.
(2) specific data are as follows:

The eye movement parameters of AD and PD patients are significantly different from those of the normal control group under the four aforementioned behavioral paradigms. In the data analysis, in addition to comparing the eye movement parameters of the same age subjects under the same eye movement task and identifying abnormal eye movements, we also took the eye movement parameters during the pro-saccade task of each subject as baseline. The baseline reference was compared with same eye movement parameters in the other three kinds of tasks and the relative changes of each subject's eye movement parameters could reflect the specific cognitive function of the brain. By analyzing and comparing the execution of the above-mentioned four types of saccade tasks (for example, error rate, saccade latency, saccade velocity, saccade accuracy), the subjects' vision, movement, and cognitive status can be understood.

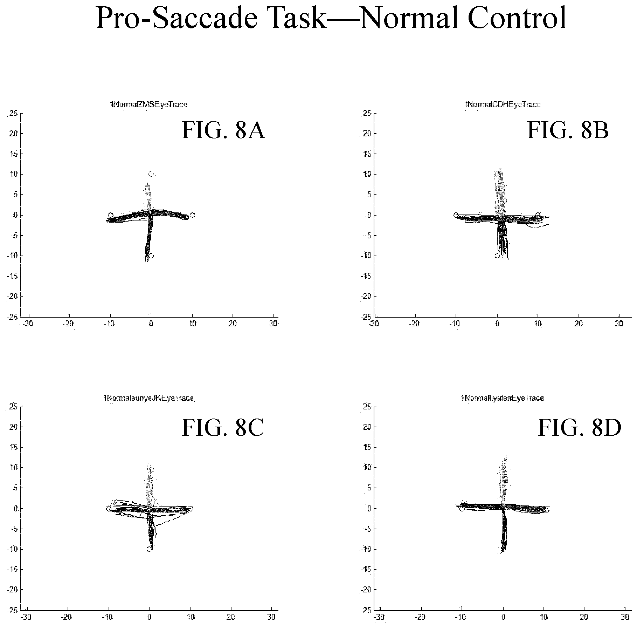

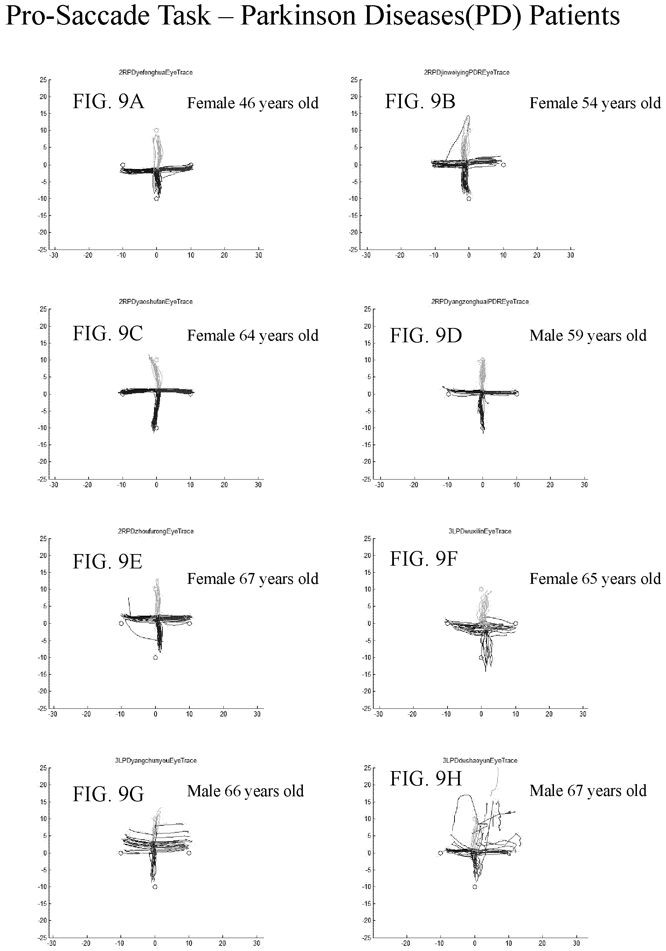

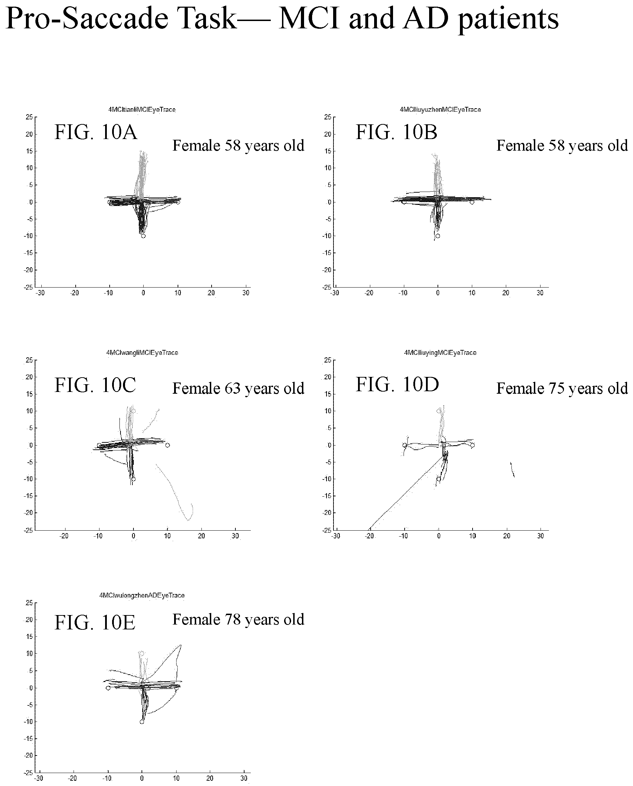

Taking three subjects as examples, among the saccadic traces under the four behavioral task paradigms, FIGS. 8 through 10 show the saccadic traces of the subjects (normal, PD, and AD) under the pro-saccade task. The pro-saccade task is mainly used to determine basic vision-saccade information processing function; different colors indicate four different saccade directions. Compared with normal controls, the saccadic traces of patients with PD and AD are disordered, especially in the patient with PD.

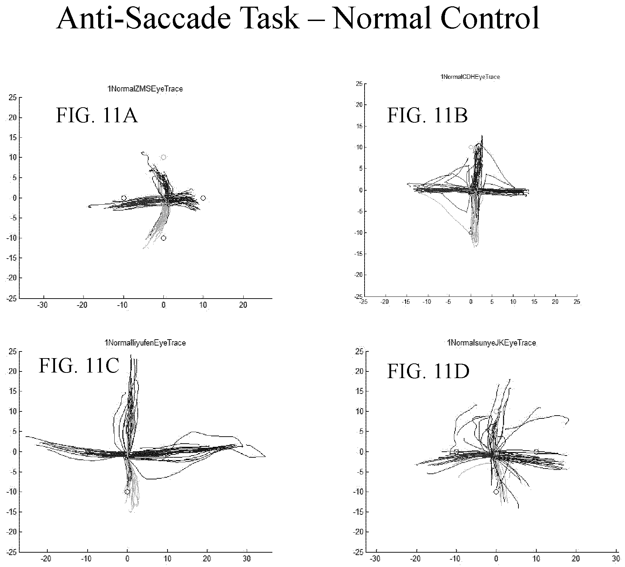

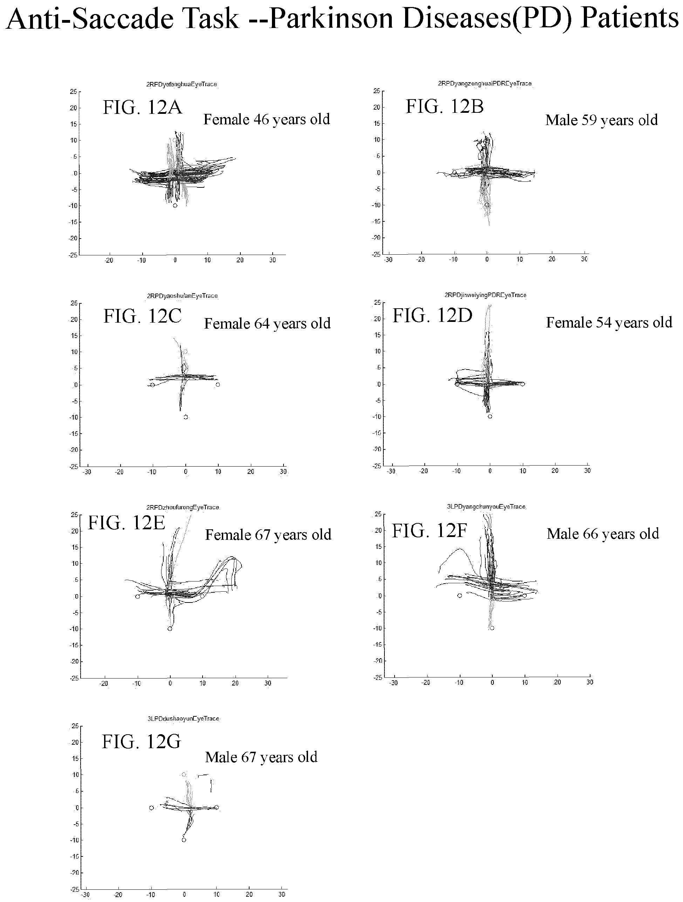

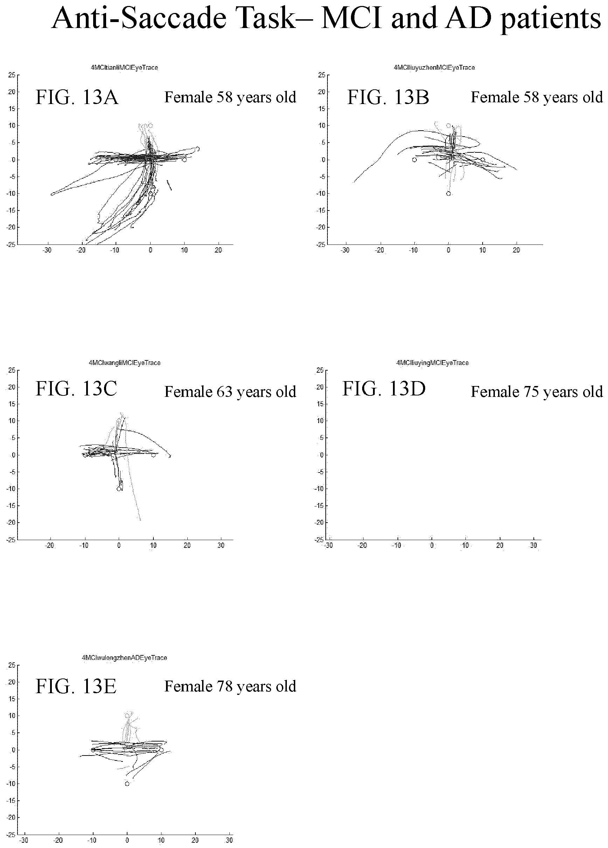

FIGS. 11 through 13 show the saccadic traces of subjects (normal, PD, and AD) under the anti-saccade task. The anti-saccade task is mainly used to detect the brain's ability to suppress reflex motions; different colors indicate four different saccade directions. Compared with normal controls, the saccadic traces of patients with PD and AD are disordered, especially in the patient with AD

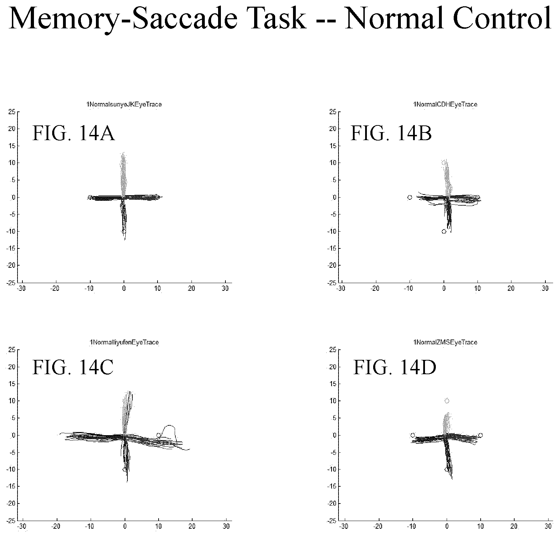

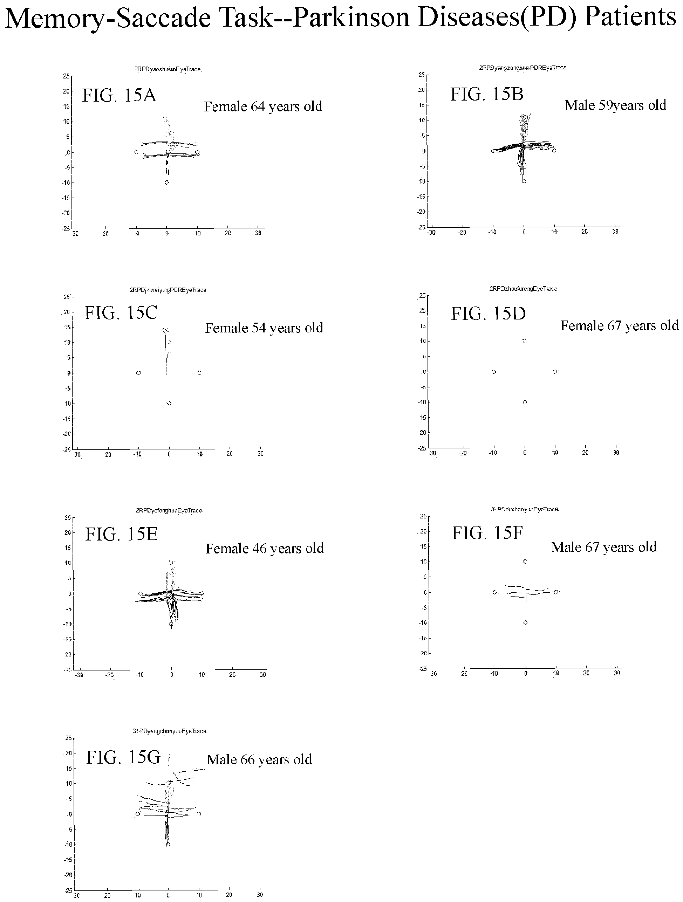

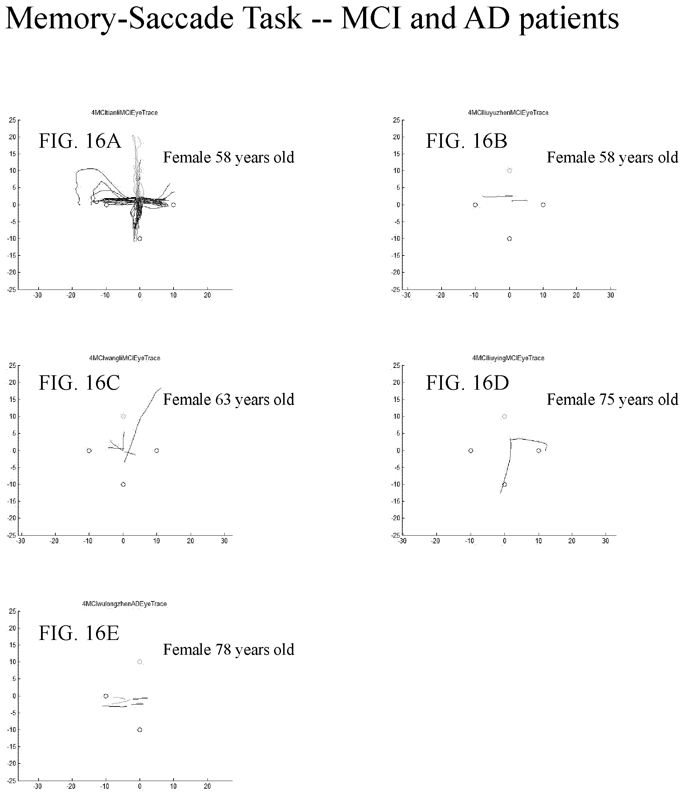

FIGS. 14 through 16 show the saccadic traces of subjects (normal, PD, and AD) under a memory-saccade task. The memory-saccade task is mainly used to determine the brain's function of short-term (working) memory; different colors indicate four different saccade directions. Compared with the normal control, the saccadic traces of the PD patient are disordered, and the AD patient cannot perform this task (very high error rate).

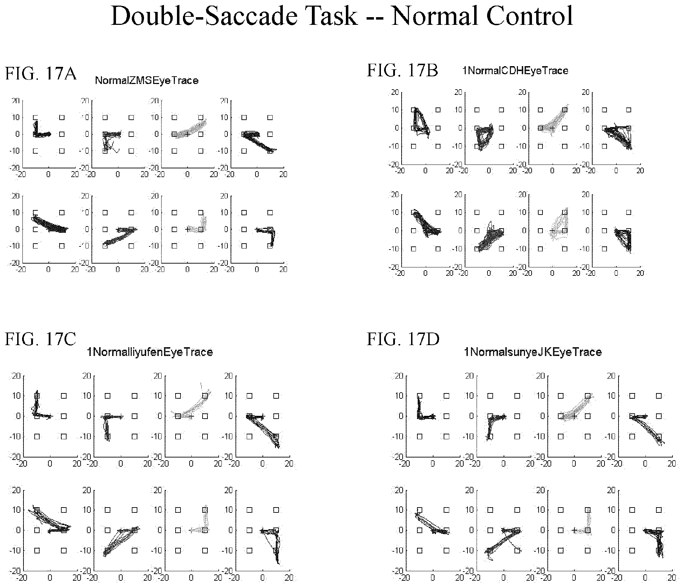

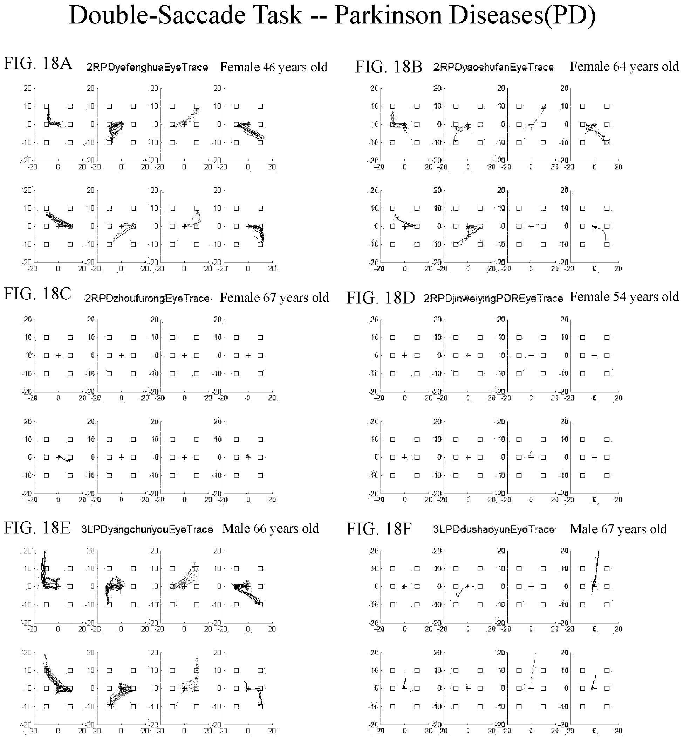

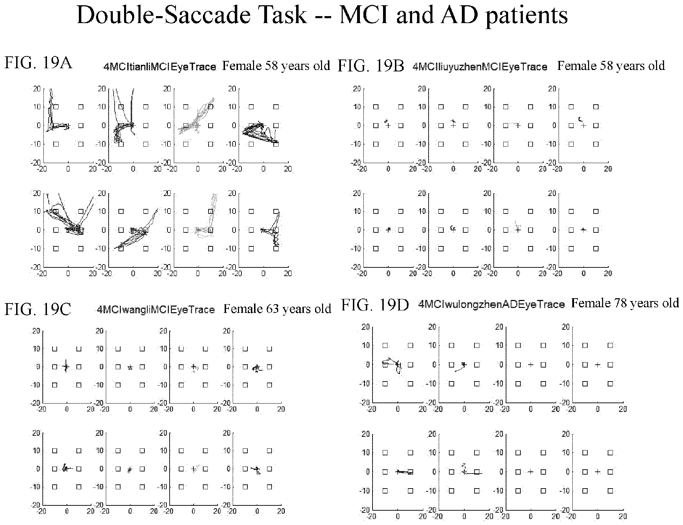

FIGS. 17 through 19 show the saccadic traces of three subjects (normal, PD, and AD) under a double-saccade task. The double-saccade task is mainly used to determine the spatial cognitive ability of the brain; different colors indicate different saccade directions. Compared with the normal control, the saccadic traces of the PD patient are disordered, and the AD patient cannot perform this task (very high error rate).

### Comprehensive error rate under four behavioral task paradigms:

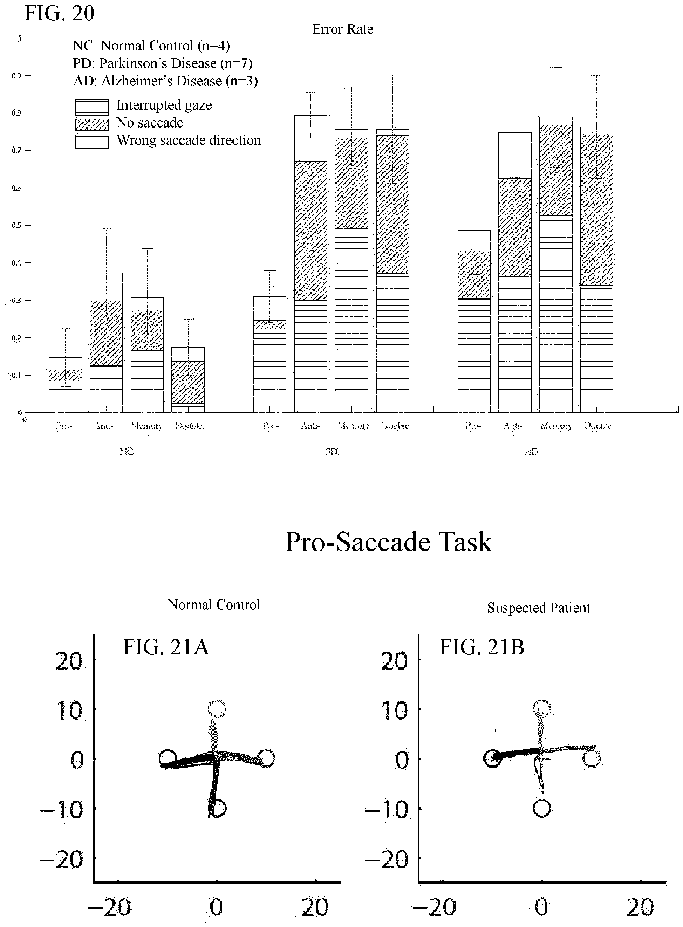

FIG. 20 shows the group mean error rates under the four tasks of subjects in the normal, PD, and AD groups. Compared with the normal control group, PD and AD patients have significantly higher error rates under the four tasks. In both the PD and AD groups, the total error rates were both more than doubled compared with the normal group, especially for AD group. From the data in Table 2, it is apparent that interrupted gaze has a greater contribution to the error rate in the disease groups, and the error rate of interrupted gaze has increased most in the disease groups in the memory-saccade task, which means the memory-saccade task is more sensitive to determine PD or AD, followed by the double-saccade task and the anti-saccade task. Therefore, these four saccade tasks are not randomly generated. Instead, the applicant determined and considered the error rate of different tasks and the vision and modules composed of different saccade tasks in the experiment, and finally formed these tasks through multi-aspect exploration and coordination. Products produced using this behavioral paradigm, such as testing devices, can achieve the function of more sensitive and accurate screening of neurological diseases.

**Table 2**

| | Error Rate | | | | |
|---|---|---|---|---|---|
| | Interrupt gaze | | No saccade | Wrong direction saccade | Total error rate |
| | Normal | 0.083 | 0.030 | 0.033 | 0.147 |
| Pro-saccade task | PD | 0.223 | 0.023 | 0.064 | 0.309 |
| | AD | 0.305 | 0.128 | 0.053 | 0.486 |
| | Normal | 0.125 | 0.173 | 0.075 | 0.373 |
| Anti-saccade task | PD | 0.300 | 0.370 | 0.124 | 0.794 |
| | AD | 0.364 | 0.260 | 0.122 | 0.746 |
| | Normal | 0.165 | 0.109 | 0.034 | 0.308 |
| Memory-saccade task | PD | 0.492 | 0.240 | 0.023 | 0.755 |
| | AD | 0.526 | 0.241 | 0.022 | 0.788 |
| | Normal | 0.025 | 0.110 | 0.040 | 0.175 |
| Double-saccade task | PD | 0.372 | 0.366 | 0.018 | 0.756 |
| | AD | 0.338 | 0.404 | 0.020 | 0.762 |

### Example 3: Early detection

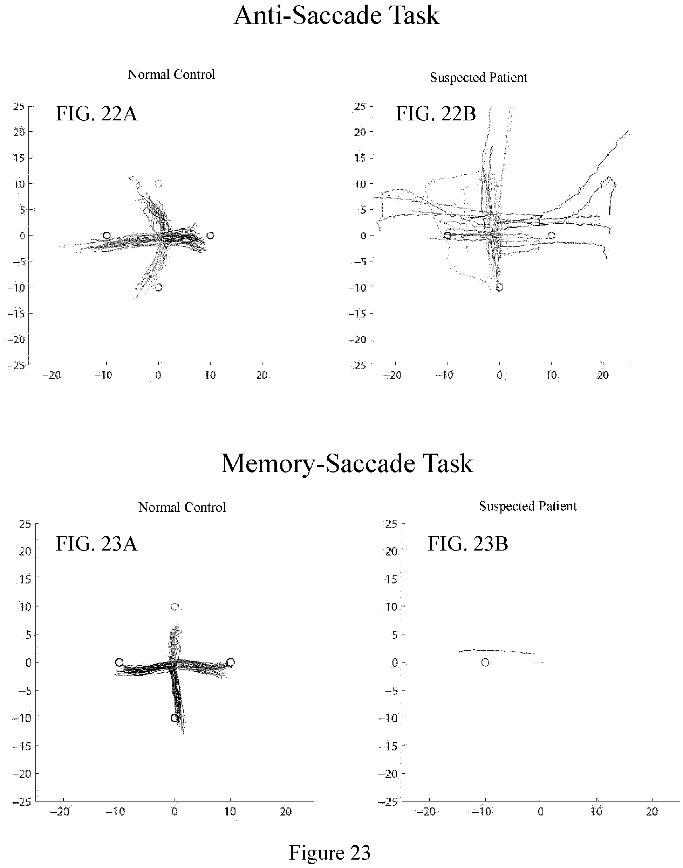

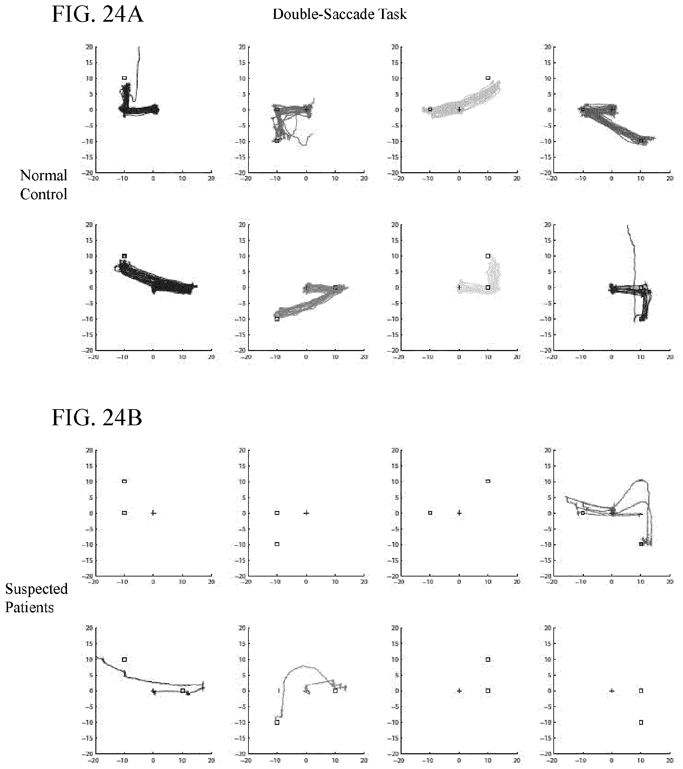

One subject sought clinical help because of paroxysmal mild tremor of the limb. Clinical examination found that the patient's other nervous system detection indicators were within the normal ranges, the brain magnetic resonance scan results were normal, and clinical diagnosis was difficult. The clinicians performed the aforementioned four eye-movement task tests for the patient and listed the experimental results in the control group (as opposed to patients with apparent PD and AD). However, when we analyzed the data, we found that many of the eye movement parameters of the subject were significantly different from those of other control subjects and were closer to the test results of PD and AD patients (FIGS. 21-24 and 27-29). Based on this, the clinician followed up the subject and carried out a number of clinical tests for 3 months, and the current initial diagnosis is essential tremor (suspected Parkinson's disease). FIGS. 21-24 show the saccadic traces of the subject and a subject from the healthy group under four saccadic tasks. Compared with the normal control, the subject's saccadic traces were significantly disordered.

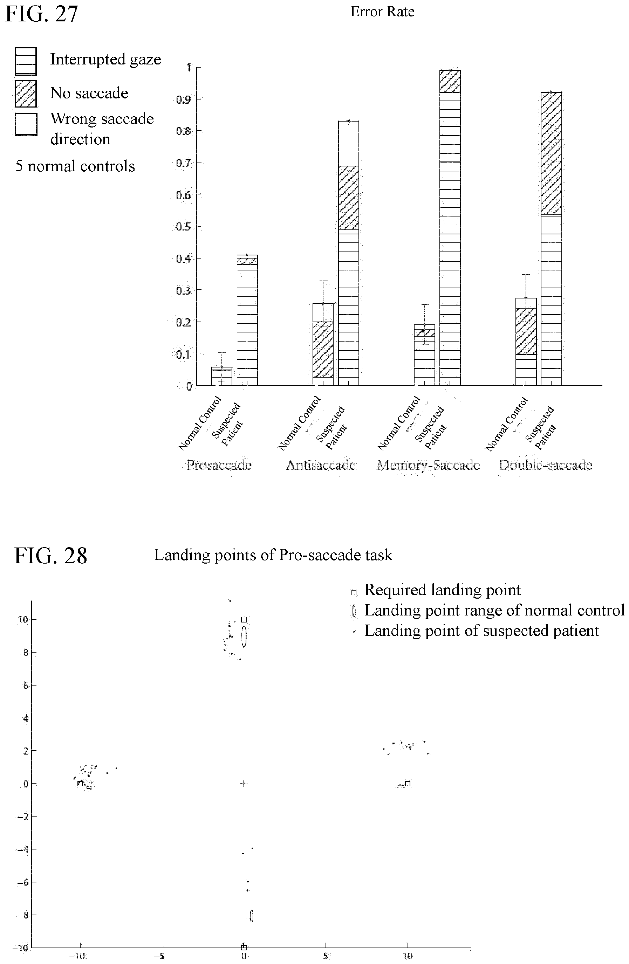

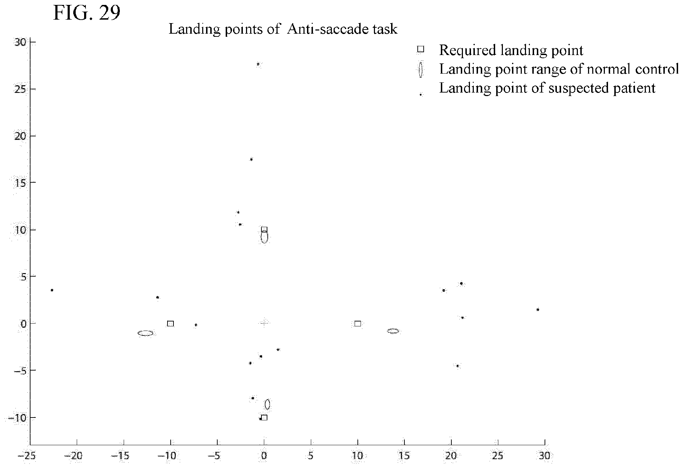

FIG. 27 shows the error rates of normal subjects and the above-mentioned subject under four tasks. Compared with the normal control group, the above-mentioned subject's error rates under the four tasks were significantly higher, and the total error rate was more than twice that of the normal subjects, and the contribution of the interrupted gaze error rate in each paradigm behavior is still the largest, further proving the core role of memory-saccade task. FIG. 28 and 29 show the saccade accuracy (saccade landing point) of normal subject and the above-mentioned subject.

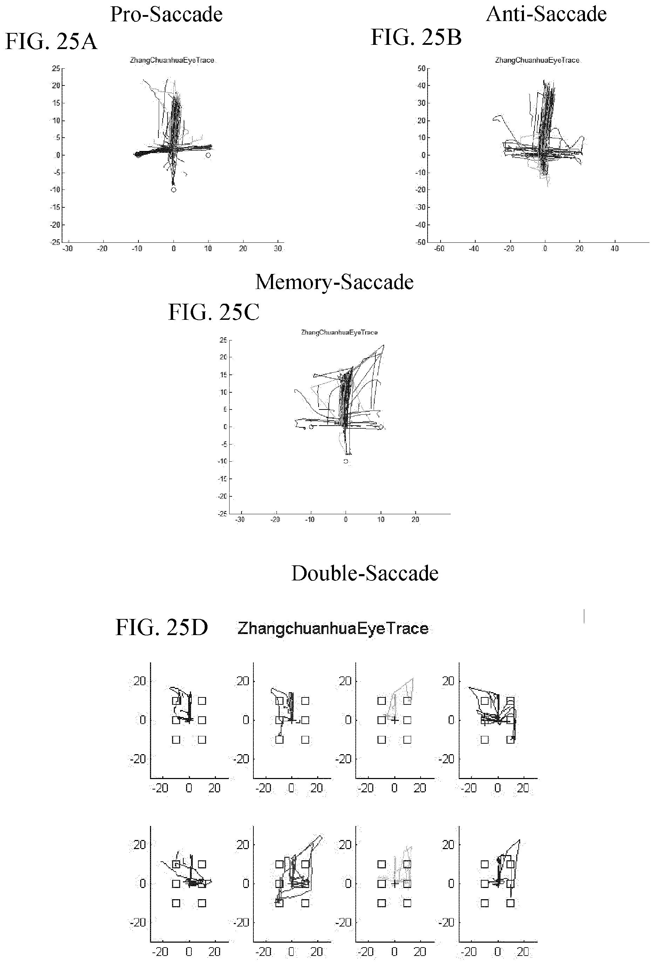

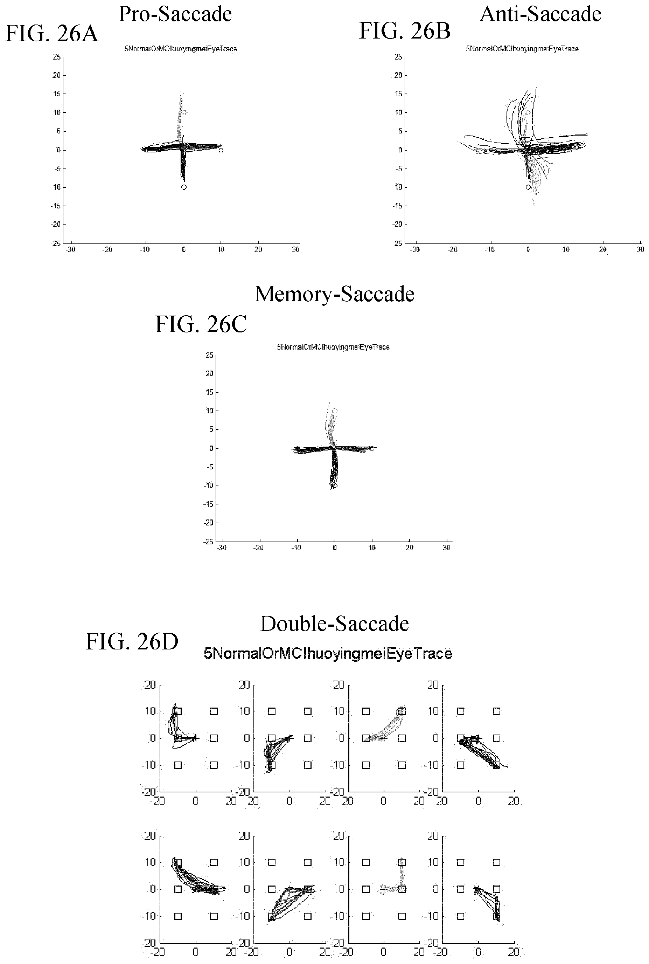

Similarly, FIGS. 25A through 25D show the saccadic traces under the saccadic tasks of the present invention of a subject who was identified as normal by classic methods. The clinical examination found that the patient's nervous system detection indicators were within the normal ranges, but after testing, the saccade tasks in the present invention indicated that the subject's saccadic traces were abnormal. After a detailed clinical review, he was finally diagnosed with essential tremor. FIGS. 26A through 26D show the saccadic traces under the saccadic tasks of the present invention of a subject who was identified as normal by classic methods. The clinical examination found that the patient's nervous system detection indicators were within the normal range, but after testing, the saccade tasks in the present invention indicated that the subject's saccadic traces were abnormal. After a detailed clinical review, he was finally diagnosed with mild cognitive impairment (MCI). The above results show that the paradigm behavior of the present invention is of great significance for the early detection of neurological diseases.

### Incorporation by reference

The entire disclosure of each patent document and scientific document cited herein is incorporated herein by reference for all purposes.

### Equivalency

The present invention may be implemented in other specific forms without departing from its basic characteristics. Accordingly, the foregoing embodiments are to be considered illustrative and not restrictive of the invention described herein. The scope of the present invention is indicated by the appended claims rather than the foregoing description and is intended to include all changes that fall within the meaning and scope of equivalents of the claims.

## Claims

1. An eye movement analysis system for detecting brain function, **characterized in that** the system comprising
vision and eye movement task module, which comprises one or more of the following tasks:
1) Pro-saccade task: The subject first looks at the gaze point, and then when the visual stimulus point appears, the subject is tasked to make a quick saccade toward the visual stimulation point;
2) Anti-saccade task: The subject first looks at the gaze point, and then when the visual stimulus point appears, the subject is tasked to make a quick saccade toward the mirror position of the visual stimulation point;
3) Memory-saccade task: The subject first looks at the gaze point, and then a visual stimulus point appears at a random position, selected among eight positions evenly distributed on the on the circle centered at the gaze point, and disappears immediately. After a period of time when the gaze point disappears, the subject is tasked to make a quick saccade to the position where the visual stimulus point once appeared;
4) Double-saccade task: The subject first looks at the gaze point. Then two visual stimulus points sequentially appear and disappear. After the gaze point disappears, the subject is tasked to make two saccades to sequentially, in the order of appearance, look at the two positions where the visual stimulus points appeared;
where the gaze point and the stimulus points do not overlap.

2. The system of claim 1, wherein the system further comprises one or more of the following modules:
a) a behavior data collection module; and
b) a data storage and analysis module.

3. The system of claim 2, wherein the behavior data collection module comprises:
a1) using computer object recognition and tracking methods to process in real-time image information collected by fast near-infrared camera in order to identify pupils and detect eye positions;
a2) eliminating in real-time the effect of head movements on the eye position monitoring.

4. The system of claim 3, wherein the frequency of the fast near-infrared camera is not lower than 1000 Hz.

5. The system of claim 3, wherein the method for eliminating the effect of head movement on eye position monitoring in real time comprises:
Place the inclinometer at the rotation point of the subject's head. When saccade and head rotation occur at the same time, the inclinometer records the current eye movement angle β and the head movement angle γ. Subtract γ from β to obtain the true saccade angle α.

6. The system of claim 5, wherein a response frequency of the inclinometer is not lower than 200 Hz.

7. The system of claim 2, wherein the data storage and analysis module comprises:
b1) establishing saccade database based on saccade behavior paradigms;
b2) establishing a computational model and analyzing eye movement parameters.

8. A brain function testing device using the system according to any one of claims 1 to 7.

9. The device of claim 8, wherein the device is used to monitor or test brain function or neurodegenerative diseases.

10. The device of claim 9, wherein the neurodegenerative diseases comprise different severity of Alzheimer's Disease, Parkinson's Disease, Huntington's Disease, cerebellar atrophy, etc.
